# EUROPEAN PATENT SPECIFICATION

(11) **EP 0 451 972 B1**
(45) Date of publication and mention of the grant of the patent: **29.05.1996**
(21) Application number: 91302396.6
(22) Date of filing: 20.03.1991
(51) Int. Cl.: A61K 47/48, A61K 7/28

(54) **Utilization of enzymes**
Verwendung von Enzymen
Utilisation d'enzymes

(30) Priority: 21.03.1990 GB 9006329; 05.10.1990 GB 9021671
(43) Date of publication of application: 16.10.1991
(73) Proprietor: UNILEVER PLC, London EC4P 4BQ (GB); UNILEVER N.V., NL-3013 AL Rotterdam (NL)
(72) Inventor: Beggs, Thomas Stewart, Elms Farm, Bedford MK41 0DN (GB); Davis, Paul James, Felmersham, Bedford MK43 7EX (GB)
(74) Representative: Ford, Michael Frederick

(56) References cited:
- WO-A-88/01178
- WO-A-90/03185
- WO-A-91/00112
- FR-A- 2 651 433
- US-A- 4 578 265
- STN FILE SERVER & FILE MEDLINE, AN=81192354; R.B. JOHNSON, Jr. et al.: "Comparison of glucose oxidase and peroxidase as labels for antibody in enzyme-linked immunosorbent assay", & J. IMMUNOASSAY, (1980), 1(1), 27-37
- SNT FILE SERVER & FILE CA, CHEMICAL ABSTRACTS, vol. 105, no. 17, abstract no. 145834w, Columbus, Ohio, US; J. PENE et al.: "In vitro cytolysis of myeloma tumor cells with glucose oxidase and lactoperoxidase antibody conjugates", & BIOCHEM. INT., 13(2), 233-43

## Description

### FIELD AND BACKGROUND OF THE INVENTION

This invention relates to the utilization of enzymes to perform a desired function, and to products for the purpose. An example of such a function is to attack species occurring in the oral microflora. Another is to attack tumour cells.

This invention entails use of two enzymes, one of which generates an intermediate product which is a substrate for the other enzyme. The latter enzyme converts the intermediate into an agent which is active against a target.

One possibility for the former enzyme is glucose oxidase. This catalyses the oxidation of glucose to gluconic acid by molecular oxygen, producing hydrogen peroxide in the process.

The other enzyme may then be a peroxidase, which functions to convert hydrogen peroxide into more potent oxidised species e.g. by reaction with halides such as iodide ion to produce hypoiodite or by reaction with thiocyanate to produce hypothiocyanate.

Hydrogen peroxide does display cytotoxicity but is rapidly decomposed in vivo. Oxidised halide species produced by peroxidase possess even greater toxicity but have an even shorter life in vivo.

### SUMMARY OF THE PRIOR ART

The use of enzymes to effect cell killing has already been proposed, and some of these proposals have sought to make use of a two-enzyme system.

Knowles et al, Journal of Clinical Investigation 52 1443 (1973), have described use of glucose oxidase chemically conjugated to antibodies capable of binding to target cells, thereby targeting the cell killing activity against those cells which it is desired to eliminate selectively. They provided peroxidase and halide in solution, and were able to demonstrate killing of bacterial cells.

Okuda et al, Infection and Immunity 27 690 (1980), have described the use of xanthine oxidase (which at least predominantly produces superoxide rather than peroxide) and lactoperoxidase chemically conjugated to antibody able to bind to target cells.

Over a period of 90 minutes, in vitro, they achieved a reduction of live Candida albicans cells, in vitro, which was between one and two orders of magnitude better than was achieved with lactoperoxidase in solution.

Pene et al Biochemistry International 2 233 (1986) discloses the killing of murine myeloma cells utilising rabbit antibodies which bind to them, and glucose oxidase and lactoperoxidase, both conjugated to goat anti-rabbit antibodies which bind to the rabbit antibodies.

### SUMMARY OF THE INVENTION

In the present invention one or more vehicles contain, in the same vehicle or distributed between a plurality of vehicles:
i) at least one of two enzymes which are an enzyme for generating an agent active against a target, and a second enzyme for generating an intermediate which is a substrate for the first enzyme,
ii) linking means attached or attachable to both enzymes to couple the enzymes to each other, at least at the time of use, and thereby form a complex containing the two enzymes linked together otherwise than through the target or through a whole antibody which binds directly to a target cell.

By linking the two enzymes, one enzyme can more readily use the intermediate produced by the other enzyme so enhancing their effectiveness.

An aspect of the invention resides in a product comprising the above-mentioned vehicles. Another aspect is a method of attacking cells by administration of the vehicles.

The linking means included in the product is able to attach, or is attached already, to the enzymes, so as to form a link between them without incorporating anything external to the product as part of the link. Preferably the linking means is something other than chemical conjugation of both enzymes to a single whole antibody.

Both enzymes may be included in the vehicle or vehicles (not necessarily in the same vehicle). Alternatively one of the enzymes may be a material which is present in vivo, for instance in the general vicinity of the intended target site and the linking means serves to couple that enzyme to the other. This would concentrate that enzyme into proximity with the other enzyme.

The enzymes which are used may be an oxidase which generates hydrogen peroxide plus a peroxidase which uses this as substrate e.g. to form hypohalite. A peroxidase may also use peroxide to convert thiocyanate to hypothiocyanate. Glucose oxidase is particularly envisaged. Another possibility is galactose oxidase, which could use as its substrate the galactose which occurs naturally in yoghurt.

Horseradish peroxidase is commercially available and could be used in conjunction with either of these oxidases. A further possibility is lactoperoxidase.

Various applications of the invention are envisaged, although the invention is not limited to these. One application which is particularly envisaged is to attack species of the supragingival oral microflora. For this and other applications the vehicle(s) preferably contain means for attaching the complex containing the linked enzymes to a target site.

The oral microflora is a complex ecosystem which contains a wide variety of microbial species. One of these species may be selected as the target site. However, the effect of targeting to one species will be to attack both that species and other species which occur in close proximity to it. Thus, by delivering to one species which occurs in dental plaque, cytotoxic agents will be delivered to the plaque and will act against all the species which occur together in the plaque, including those responsible for plaque formation. Extracellular dextran produced by such organisms could itself be used as a target site.

One possible target site is Streptococcus mutans. This has been identified as an important contributor to dental plaque, and has been shown to be capable of inducing clinical caries lesions in germ free animals when established as a mono-infection. S. mutans has the ability to utilise dietary carbohydrate for the synthesis of an insoluble polysaccharide matrix, facilitating attachment to, and colonisation of, hard surfaces, as well as production of acids capable of the dissolution of enamel. These characteristics have been identified as important virulence determinants. Although other species and genera have also proved capable of both acid and plaque production, or even of caries initiation in the germ free animal, S. mutans is widely recognised as at least one significant cause of tooth decay because of the scale of its acid and polysaccharide production.

Other species which may be selected as the target species are S. sanguis, A. viscosus and A. naeslundii. These are all present in dental plaque as a substantial proportion of the species normally found in dental plaque. Because of frequent occurrence, these three may be preferred as target site.

Another application is to attack species of the subgingival microflora responsible for periodontal disease. The target species could well be Bacteroides gingivalis.

A possible cosmetic application is the reduction of stain on teeth. In this application enzymes are used which produce a material with a bleaching function, such as hypohalite ion. The target site is at the tooth surface where staining may be present.

For any oral application (dental care) it would be necessary for the vehicle(s) in the product to be acceptable to enter the mouth, e.g. vehicles suitable for topical application in the mouth.

Another application is to attack human tumour cells, notably in bone marrow which has been removed temporarily from the body of a patent undergoing radiotherapy.

### BRIEF DESCRIPTION OF THE DRAWINGS

The invention may be implemented in various ways. Some of these are illustrated in the accompanying drawings which are all schematic diagrams of linking arrangements. G.Ox denotes glucose oxidase. HRP denotes horseradish peroxidase. PEI denotes polyethyleneimine.

In the drawings:
Fig. 1 shows two enzymes linked by means of polyethyleneimine;
Figs. 2 and 3 each show two enzymes linked together by means of antibodies;
Figs. 4 to 8 each show one of the above complexes attached to a target;
Fig. 9 shows two enzymes linked together and attached to a target by means of antibody fragments;

### DETAILED DESCRIPTION OF EMBODIMENTS

This invention requires two enzymes to be linked together. The complex containing the enzymes which are linked may be attached to a target. Attachment is preferably accomplished by means of an antibody or antibody fragment which binds to the target site.

In this invention, the linking means extends between the enzymes and couples the enzymes together otherwise than through the target site (which may be a cell) or through a single whole antibody which binds directly to the target site. It is possible to avoid subjecting a target-specific antibody to artificial chemical reactions used to effect conjugation of enzymes to the antibody through covalent bond formation. Secondly, linking the enzymes otherwise than through the target site or a target-specific antibody can make it easier to control the distribution of enzymes and get the two kinds of enzyme in proximity to each other, so that the intermediate which is the product of one enzyme is generated in proximity to the other enzyme.

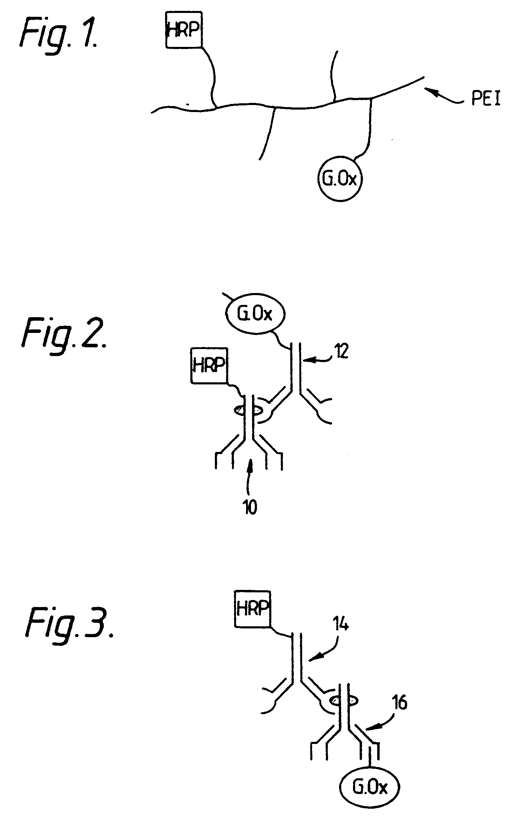

One possibility is that the linking means is a carrier material to which both enzymes are conjugated chemically by covalent bond formation. This carrier material can be a synthetic polymer having chemical functionality to enable the attachment of enzymes by chemical reaction. This is illustrated by Fig. 1. A suitable polymer is polyethyleneimine (PEI) which is a branched polymer with amino groups at the termini of the branches.

This provides a further aspect of this invention, which is two enzymes, one of which produces a substrate for the other, both conjugated through covalent bonds to a synthetic polymer.

Glucose oxidase and horseradish peroxidase are both enzymes with pendant glycosyl chains. Such enzymes can be covalently bound to polyethyleneimine by first oxidising the enzymes in aqueous solution with periodate to generate aldehyde groups in the pendant glycosyl chains. These groups will then form Schiff bases with amino groups on the polyethyleneimine, at alkaline pH (e.g. pH 9.5) after which reduction with borohydride can be used to reduce any unreacted aldehyde groups and also increase the stability by reduction of the Schiff bases.

These enzymes can also be conjugated to antibodies by the same technique.

The linking means may comprise at least one antibody (preferably other than a target-specific antibody) to which the enzymes are joined. The enzymes may each be attached or attachable to a respective antibody while these antibodies are able to bind to each other by antibody-antigen binding. An example of this is shown in Fig. 2 where the enzymes are conjugated chemically to respective antibodies from different species, one of which is an antigen for the other.

In Fig. 2, numeral 10 denotes rabbit anti-bovine immunoglobulin to which horseradish peroxidase is conjugated. Numeral 12 denotes sheep anti-rabbit immunoglobulin conjugated to glucose oxidase. This binds to antibody 10 so linking the enzymes.

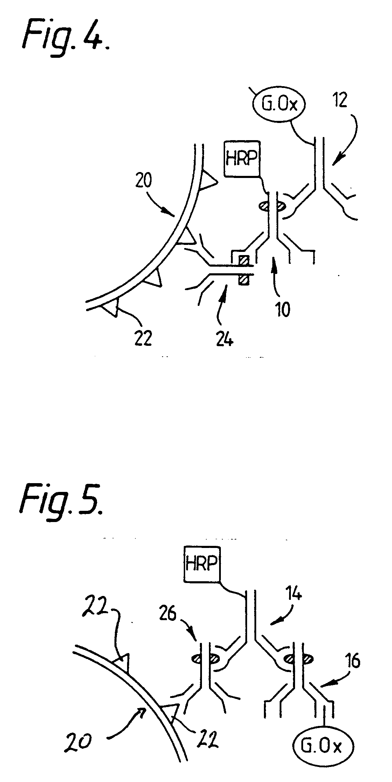

A variation is shown in Fig. 3. Horseradish peroxidase is conjugated chemically to goat anti-rabbit immunoglobulin. The goat antibodies 14 bind to rabbit anti-glucose oxidase antibodies 16 which bind to glucose oxidase. Thus the two enzymes are linked together through the antibodies 14,16.

Two linked enzymes, connected together in accordance with this invention can display a cell killing activity against a target even if there are no deliberate measures to attach the linked enzymes to the target. This can arise simply because bringing the enzymes together enhances activity. Also the complex of enzymes and linking means may have an inherent affinity for the site of the target.

However, it is preferred that the linked enzymes are coupled to the site of the target. The complex consisting of the two enzymes and linking means may be attached or attachable to an antibody or antibody fragment able to bind to the target site. Desirably this attachment is by antibody-antigen binding, which can avoid chemical conjugation to target-specific antibody.

If the linking means includes one or more antibodies, one of these can have the ability to bind to target-specific antibody which is included in the vehicle(s). This is illustrated by Figs. 4 and 5.

In Fig. 4 numeral 20 denotes a target which is S. mutans and numeral 22 denotes antigenic sites on the cell surface. Numeral 24 denotes bovine anti-S. mutans antibody which is included in the overall product. This binds to the target and the rabbit anti-bovine antibody 10 of the complex shown in Fig. 2 binds to the bovine antibody 24.

Similarly in Fig. 5 the antibody 14 of the complex of Fig. 3 binds to rabbit anti-S. mutans antibody 26 which binds to the target 20.

Attachment to a target through a sequence of antibodies is the subject of our co-pending application of even date herewith.

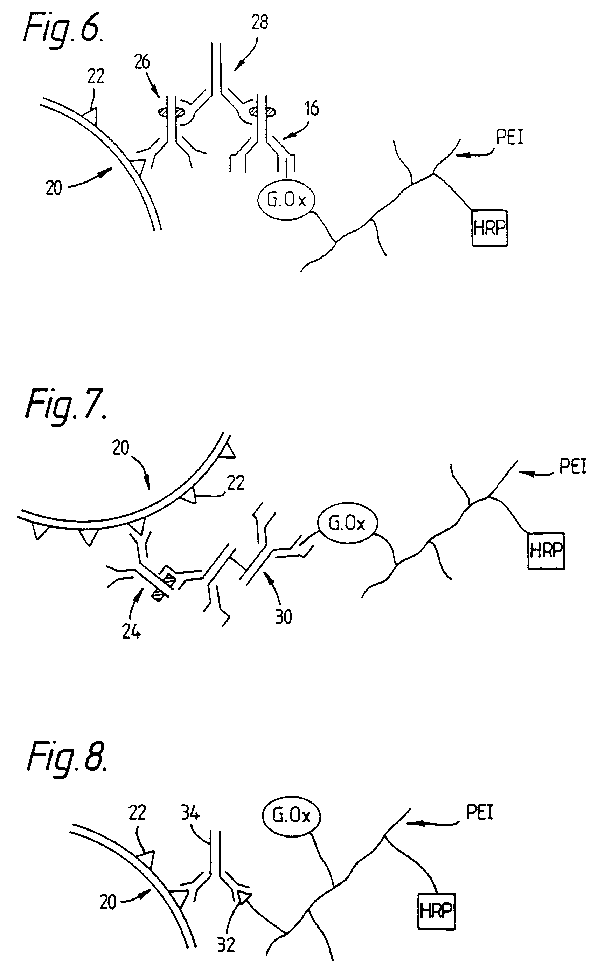

If the linking means is a synthetic polymer as illustrated in Fig. 1, a complex of such polymer with both enzymes bound to it through covalent bonds could be attached to the target site in various ways. One possibility is for the product to include an antibody to one of the enzymes, an antibody to the target and a further antibody able to form a bridge by binding both to this and to the antibody which binds to the target site. This is illustrated by Fig. 6 where many numerals have the same significance as in Fig. 5. Numeral 28 denotes goat anti-rabbit immunoglobulin.

Another possibility is for the product to include a double antibody conjugate with two specificities which has the ability to bind to one of the enzymes and also to bind to antibody which binds to the target. Double antibody conjugates are known per se. This arrangement is illustrated by Fig. 7. Numeral 24 denotes bovine antibody to the S. mutans target 20. Numeral 30 denotes a double antibody conjugate of anti-bovine and anti-glucose oxidase antibodies.

A further possibility is for the polymer to have antigenic sites characteristic of the target covalently bound to it. Antibody able to bind to the intended target would also bind to these antigenic sites on the polymer and in that way couple the polymer to the target site. This arrangement is illustrated by Fig. 8, where numeral 32 denotes an antigen attached to polyethyleneimine and numeral 34 denotes an anti-target antibody binding to the target 20 and to the antigen 32.

Linking of enzymes and binding to a target may be accomplished by means of antibody fragments. A preferred arrangement utilises an antibody fragment to bind to a target site and further fragments to bind enzymes to the first fragment.

The first antibody fragment which binds to a target site may be an Fv fragment of an antibody to the desired target. Such a fragment contains only the variable domains of light and heavy chains of an antibody. The fragment could possibly be an F(ab)₂ fragment which would provide two combining sites. It might alternatively be as little as a single variable domain of one chain of an antibody.

Techniques for efficient production of biologically active antibody fragments in E. coli were described by A Skevia and A. Pluckthun (1988), Science, 240, 1038; M. Better et al, (1988), Science, 240, 1041; and E.S. Ward et al, (1989), Nature, 341, 544. The cloning and expression of genes encoding antibody fragments in E. coli is also described in published European application EP-A-368684 (Medical Research Council).

We prefer that a first antibody fragment has an additional peptide chain appended to it, and further fragments bind the enzymes to this peptide chain. Bacteria can be made to produce a variable domain with an extra peptide chain already attached to the C-terminus of the domain. This can be achieved by standard genetic techniques. For example, a gene encoding a variable domain can easily be lengthened by means of site directed mutagenesis techniques, using in vitro synthesised oligonucleotides which encode the peptide to be appended to the variable region. Site directed mutagenesis is now a widely used technique, and adequate protocols can be found in several published books, for example in Sambrook et al, (1989), Molecular Cloning, 2nd edition, Cold Spring Harbour Laboratory Press, New York.

An attached extra peptide provides a very convenient "handle" for the attachment of the therapeutic agent.

Further antibody fragments which bind enzymes to the first fragment are preferably a second antibody fragment able to bind to an enzyme by antibody-antigen binding, and an F(ab)₂ fragment (which is bivalent) able to bind to the first and second antibody fragments, especially to antigenic peptides appended to the first and second antibody fragments.

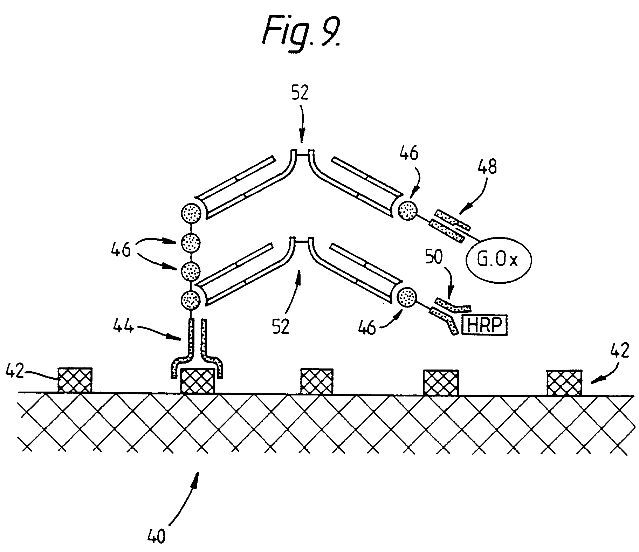

Fig. 9 illustrates a preferred arrangement in which antibody fragments are utilised.

For attaching to the target 40 which has antigenic sites 42 there is an Fv antibody fragment 44 with several repeats of a peptide 46 appended to the distal (c-terminal) end of one of the two chains in the Fv fragment 44.

Glucose oxidase (G.Ox) and horseradish peroxidase (HRP) are each bound by a respective Fv fragment 48,50 with specificity for the enzyme concerned, and with a single repeat of the same peptide 46 appended to one chain of the Fv fragment.

The peptides 46 appended to the anti-enzyme Fv fragments 48,50 become linked to peptides 46 on the anti-target Fv fragment by F(ab)₂ fragments 52 which bind specifically to these peptides. Since the F(ab)₂ fragments are divalent they can form a bridge attaching an anti-enzyme fragment, with attached enzyme, to the anti-target fragment 44.

Antibodies used in this invention may be polyclonal or monoclonal. Where an antibody of one specificity and an antibody of a different specificity are used together, it is possible that one antibody would be monoclonal while the other antibody was polyclonal.

If a plurality of polyclonal antibodies are used, it may be found desirable to distribute the enzymes and antibodies between more than one vehicle in the product , so that the full complexes of both enzymes and antibodies do not form until the time of use. We have found that during storage, large complexes with polyclonal antibodies are prone to suffer a reduction in their ability to bind to a target site.

Distribution of constituents of the complex between a plurality of vehicles may be unnecessary if monoclonal antibodies are employed. In general, monoclonal antibodies would form smaller complexes and during storage would be expected to retain their activity better than complexes formed with polyclonal antibodies. This is also true when antibody fragments are used. An advantage of antibody fragments is that the complexes which form by antigen - antibody binding are fairly small and more stable than complexes with whole antibodies.

When a product has the enzymes and one or more antibodies distributed between two vehicles, one of them could contain antibody able to bind to the target while the other vehicle could contain the enzymes and means to link them together, possibly as a preformed complex.

A product comprising a vehicle or vehicles containing enzymes, means to link them and/or one or more antibodies or antibody fragments could take a number of forms. If the target site is in the mouth, possibilities include mouthwash, toothpaste and a lozenge which will dissolve in the mouth. These forms of product could be used even when a plurality of vehicles are needed. For instance the product could be a two-component mouthwash which the user mixes immediately before use.

It could be a toothpaste having two components stored in the toothpaste container in such a way that they are kept separate or at least do not mix but are dispensed together and mix in the mouth of the user. Such two-component toothpaste products are known per se.

Another possible form of product providing a plurality of vehicles would be a lozenge to be sucked in the mouth, with the various materials contained in separate regions of the lozenge.

Two vehicle forms could be used in combination as a way to provide a plurality of vehicles, e.g. toothpaste whose use is followed by a mouthwash or a lozenge.

The product may include some or all of the substrates for the enzymes, apart from the enzymatically generated intermediate, or it may rely on some or all enzyme substrates being present at the target site. Thus where glucose oxidase is used and the target site is in the mouth, the product could rely on dietary glucose as the enzyme substrate or it could itself incorporate glucose provided this was separate from the glucose oxidase.

### EXAMPLES

The invention is further explained by the following experimental Examples, demonstrating effectiveness of the system in vitro.

In the Examples "PBS" denotes phosphate buffered saline having pH 8 unless otherwise stated.

Dilutions are expressed in the form "1/n" signifying that a quantity of starting solution was mixed with diluent to give a solution in which the concentration was one n'th that of the starting solution.

### Example 1 : Part 1 - Materials employed

Horseradish peroxidase conjugated to rabbit anti-bovine immunoglobulin was a commercial product (Sigma).

Glucose oxidase conjugated to sheep anti-rabbit immunoglobulin was a commercial product (Serotec).

Bovine anti-S. mutans was prepared as follows: S. Mutans was cultured overnight in Todd-Hewitt broth. The culture was killed with heat, mixed with sterile saline and fumed silica (Gasil) adjuvant to yield a solution containing 0.1g/ml Gasil and 10⁸ cells/ml. 2ml of this solution was injected intramuscularly into a calf. A similar injection was given three weeks later and after two more weeks antisera was extracted from whole blood. Rabbit anti-bovine immunoglobulin (used for comparisons) was also a commercial product (Sigma).

All reagent solutions, and PBS used for washing contained 0.15% v/v of the surfactant Tween 20 (polyoxyethylene (20) sorbitan monolaurate).

### Example 1 : Part 2 - Procedure

An experimental procedure was carried out in which S. mutans was exposed to bovine anti-S. mutans, then to materials to form a cell killing complex attached to the S. mutans through the bovine antibodies thereto.

Controls were carried out in which steps of the procedure were omitted or varied.

The procedure was a series of steps as follows:
1. 10ml aliquots of a culture of S. mutans in Todd Hewitt broth were transferred to sterile McCartney bottles. The bacteria were centrifuged into a pellet, washed 3 times with PBS at pH 8 and resuspended into 9ml PBS.
2. 1ml of bovine anti-S. mutans suspension was added. This suspension was a 1/10 dilution of whole serum in PBS at pH 9, sterilised by filtration. Alternatively, 1ml of PBS at pH 8 was added as control.
3. The suspension was incubated at room temperature for 1 hour, then the cells were centrifuged into a pellet and washed three times with PBS and resuspended in 9ml PBS as before.
4. 1ml of filter-sterilised horseradish peroxidase/rabbit anti-bovine conjugate, at 1/10 dilution in PBS at pH 8, was added giving a final dilution of 1/100. After 20 minutes incubation at room temperature the cells were centrifuged into a pellet, washed 3 times with PBS at pH 8 and resuspended in 9ml PBS as before.
   In a control, 1ml of PBS was added in place of the conjugate. In comparative experiments the conjugate was replaced with rabbit anti-bovine immunoglobulin.
5. 1ml of filter sterilised glucose oxidase/sheep anti-rabbit conjugate, at 1/10 dilution in PBS at pH 8 was now added, giving a final dilution of 1/100 after addition. Again, the suspension was incubated at room temperature for 20 minutes, then the cells were centrifuged into a pellet and washed 3 times with PBS.
6. The cells were resuspended in 10ml of filter sterilised PBS at pH 6.5 containing 15µg/ml potassium iodide and 5% w/v D-glucose.
7. The resulting suspension was incubated at 37°C for 24 hours. 0.5ml samples were taken immediately on mixing, and at intervals. The bacteria in each sample were separated by centrifugation into a pellet which was washed three times with PBS. The viable cells in each sample were assayed by the method of Miles, Misra and Irwin J. Hygiene 38, 732-749, (1938). The combinations of materials added, and the counts of viable cells are set out in the following Table.
8. Samples of the suspension produced in step 6 were assayed colorimetrically for the simultaneous presence of both enzymes bound to the cells. The cells were centrifuged at 4000rpm for 5 minutes and then washed 3 times by resuspending the pellet in 3ml of PBS, and centrifuging again. After the last centrifugation the cells were resuspended in 0.5ml of PBS containing 100mM glucose and 1µg/ml of tetramethyl benzidine. This system develops colour only where glucose oxidase and peroxidase are present together. Colour was allowed to develop for 5 minutes, then stopped by addition of 50µl of 0.2M HCl. Optical densities were determined and are included in Table 1.

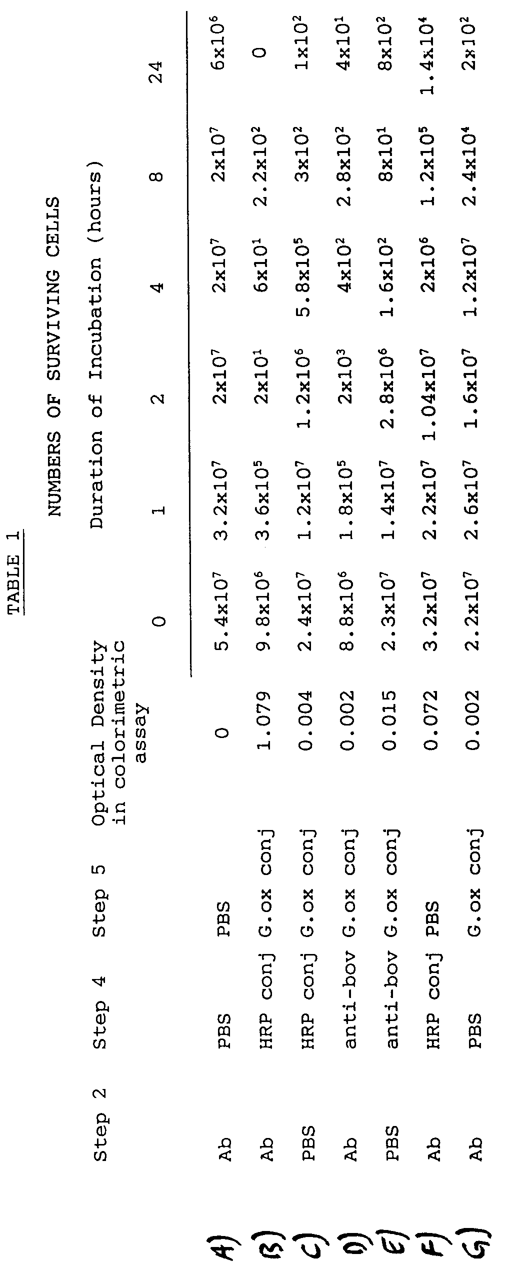

As can be seen from the Table, the cells generally survive in the presence of antibodies to them (experiment A). When glucose oxidase conjugate is present, but unable to bind to the S. mutans cells (experiment G) it displays a cell killing effect. It displays a greater cell killing effect if bound to the target S. mutans cells (experiment D). Surprisingly, horseradish peroxidase conjugate produced some cell killing without glucose oxidase (experiment F).

In experiment C both conjugates were present and able to complex together.

Experiment C shows both conjugates present and able to complex together. Thus this experiment had both enzymes present and linked together. There was cell killing and it was greater than when glucose oxidase conjugate was present, but unbound, (experiment G) or when horseradish peroxidase alone was bound to the target (experiment F).

Even greater cell killing is achieved by experiment B which contains all the components to enable both conjugates to attach together and also attach to the target cells. The number of surviving cells dropped dramatically within two hours, and eventually fell to zero.

### Example 2

The procedure of Example 1 was repeated, using two different dilutions of the glucose oxidase/sheep anti-rabbit conjugate. In some experiments the conjugate used was 1/10 dilution in PBS at pH 8, so that after addition the final dilution of the conjugate was 1/100. In other experiments the conjugate was used at 1/160 dilution, so that after addition the final dilution was 1/1600.

The combinations of materials added, the counts of viable cells, and optical densities from the colorimetric assay are set out in the following Table.

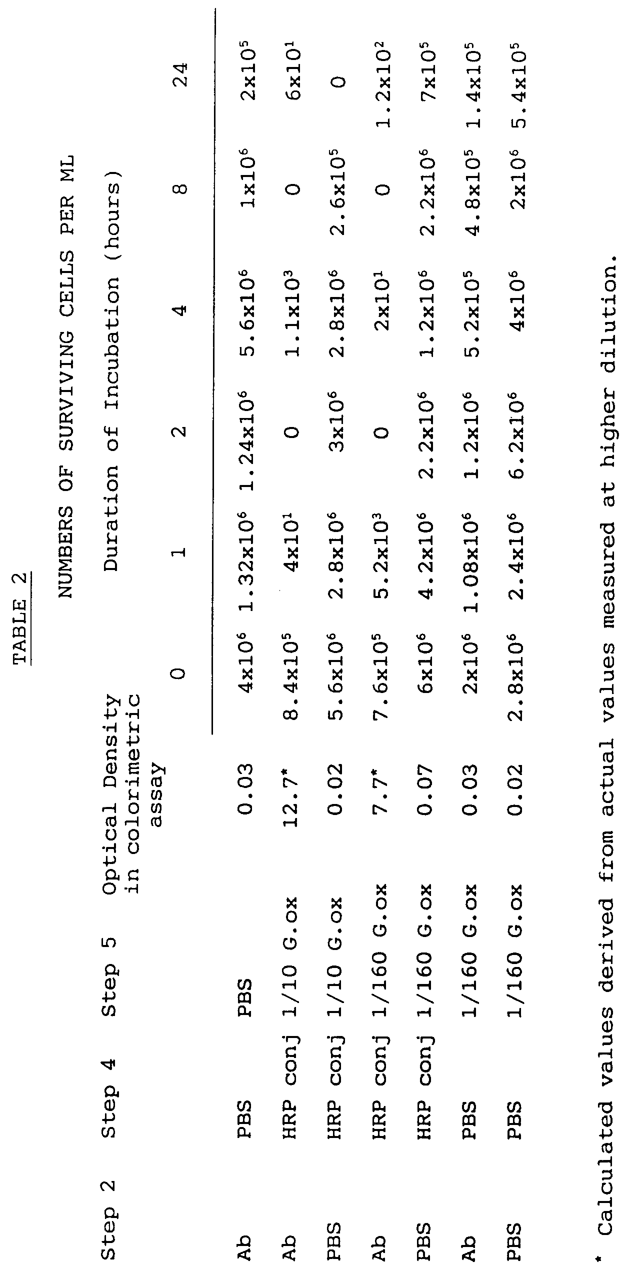

It can be seen from Table 2 that cells survive in the presence of antibodies to them (experiment A) and that the dilute glucose oxidase conjugate displays little cell killing activity in the absence of peroxidase (experiments F and G).

Experiments C and E both used linked enzymes, because there was a complex of peroxidase conjugate with glucose oxidase conjugate. In both experiments there was cell killing, although experiment E with dilute glucoseoxidase conjugate gave markedly less cell killing than experiment C using the more concentrated glucose oxidase conjugate. Much faster cell killing was observed with complex bound to the S. mutans cells (experiments B and D). The effectiveness of the bound complex with the dilute glucose oxidase conjugate (experiment D) was particularly notable.

### Example 3

This example demonstrates the covalent conjugation of glucose oxidase (G.Ox) and horseradish peroxidase (HRP) to polyethyleneimine (PEI) of molecular weight 50,000-60,000 (ex Aldrich). Covalent coupling is achieved by periodate oxidation of pendant glycosyl chains on the enzymes, to generate aldehyde groups which react with amino groups on the PEI. These chemical links (Schiff's bases) are subsequently stabilised by reduction with sodium borohydride, which simultaneously removes unused reactive aldehydes (by reduction to alcohols) to prevent further chemical coupling.

### Part 1 : Preparation of the dual enzyme-PEI (DEPEI) conjugate

### 1. Oxidation of the enzymes

G.Ox (10mg) was dissolved in 2.0ml distilled water, and then mixed with 0.2ml of a freshly prepared 0.1M solution of sodium metaperiodate in distilled water. The mixture was stirred for 20 minutes in the dark at ambient temperature, after which it was dialysed overnight at 4°C against 1mM sodium acetate/acetic acid buffer, pH 4.4 (1 litre).

HRP was oxidised in the same way, except that it was at a concentration of 2.5mg in 2ml distilled water.

### 2. Conjugation of oxidised enzymes to PEI

The pH of each oxidised enzyme solution was raised by the addition of 0.05ml of 0.2M sodium carbonate/bicarbonate buffer, pH 9.5. At this point, 0.2ml of the oxidised G.Ox solution was mixed with 0.2ml of a 100µg/ml solution of PEI in 0.01M sodium carbonate/bicarbonate buffer at pH 9.5. The mixture was kept at ambient temperature in the dark for 30 minutes. 0.2ml of the oxidised HRP was then added, and the reaction was continued for a further 2 hours (still in the dark). At the end of this stage the reaction was stopped by the addition of 0.03ml of a 5mg/ml solution of sodium borohydride in distilled water.

### Part 2 : It was demonstrated in vitro that the DEPEI conjugate would attach to S. mutans cells

A culture of S. mutans cells (as described in the earlier examples) was washed by centrifugation and resuspension (4 times) in PBS and finally resuspended in the original volume of PBS. Samples of this bacterial suspension (0.2ml) were mixed with equal volumes of PBS containing 1% bovine serum albumin and 0.15% Tween 20 (PBST/BSA), held at ambient temperature for 30 minutes, sedimented again by centrifugation and then resuspended in a solution of DEPEI conjugate diluted 1/50 in PBST/BSA (at pH 8.0).

The S. mutans cells and DEPEI conjugate were left in contact for 30 minutes at ambient temperature, after which the cells were sedimented and resuspended in PBST/BSA 3 times. An identical control sample was subjected to the same procedure, but without the addition of any DEPEI conjugate.

To demonstrate the presence of bound DEPEI on the surface of the cells, the sedimented pellet was resuspended in 0.5ml of a solution of tetramethyl benzidine (TMB, Sigma) and glucose in 0.1M phosphate/citrate buffer, pH 6.5 (TMB at 100µg/ml and glucose at 27mg/ml). This mixture was maintained at ambient temperature for 5 minutes, after which the cells were centrifuged to a pellet and a 0.2ml sample of the supernatant fluid was transferred to a microtitre plate. 0.05ml of 2M hydrochloric acid was added, then the optical density of the fluid was measured. Optical densities were:
Experimental sample : 1.07
Control (no DEPEI) : 0.01

This experiment was repeated with varying pH for the solution of DEPEI conjugate. It was found that if a pH of 5.5 to 7.5 was used the optical density rose slightly, indicating more binding. If pH was 8.5 or greater the optical density fell sharply. Changing the ionic strength by addition of sodium chloride also affected binding, indicating that the binding of DEPEI to S. mutans cells is by ionic interactions.

## Claims

1. A product comprising one or more vehicles containing, in the same vehicle or distributed between a plurality of vehicles:
i) at least one of two enzymes which are an enzyme for generating an agent active against a target and a second enzyme for generating an intermediate which is a substrate for the first enzyme,
ii) linking means attached or attachable to both enzymes to couple the enzymes to each other, at least at the time of use, and
thereby form a complex containing the two enzymes linked together otherwise than through the target or through a whole antibody which binds directly to a target cell.

2. A product according to claim 1 wherein the first enzyme is an oxidase which generates hydrogen peroxide and the second enzyme is a peroxidase.

3. A product according to claim 1 or claim 2 including both enzymes.

4. A product according to any one of claims 1 to 3 wherein the linking means comprises one or more antibodies or antibody fragments to which the enzymes are joined, at least at the time of use.

5. A product according to claim 4 wherein each enzyme is joined to a respective antibody and the antibodies bind to each other by antibody-antigen binding.

6. A product according to claim 5 wherein at least one enzyme is chemically conjugated to its respective antibody.

7. A product according to claim 4 wherein the linking means comprises at least one antibody fragment to which one enzyme is attached, at least at the time of use, and which is able to link to the other enzyme through antibody-antigen binding.

8. A product according to any one of claims 1 to 3 wherein the linking means is a carrier material to which both enzymes are chemically conjugated.

9. A product according to claim 8 wherein the carrier material is polyethyleneimine.

10. A product according to any one of claims 1 to 9 also containing an antibody or antibody fragment which is attached or attachable to the complex comprising the linking means and the two enzymes and which is able to bind to the target site.

11. A product according to claim 10 wherein attachment of the said complex to the antibody or antibody fragment able to bind to the target site is through antibody-antigen binding.

12. A product comprising one or more vehicles containing, in the same vehicle or distributed between a plurality of vehicles:
i) at least one of two enzymes which are an enzyme for generating an agent active against a target and a second enzyme for generating an intermediate which is a substrate for the first enzyme,
ii) linking means attached or attachable to both enzymes to couple the enzymes to each other, at least at the time of use, and
iii) an antibody fragment which is attached or attachable to the complex comprising the linking means and the two enzymes and which is able to bind to the target site.

13. A product according to any one of the preceding claims wherein the target is an oral bacterial species and the or every said vehicle is acceptable to be taken within the mouth.

14. A product according to claim 13 which is, or in which at least one vehicle is provided by, a toothpaste, a mouthwash or a lozenge.

15. A cosmetic method of attacking a target which comprises delivering to the vicinity thereof a product according to any one of the preceding claims.

16. A cosmetic method of attacking a target which comprises delivering to the vicinity thereof
i) at least one of two enzymes which are an enzyme for generating an agent active against a target, and a second enzyme for generating an intermediate which is a substrate for the first enzyme, and
ii) linking means attached or attachable to both enzymes to couple the two enzymes to each other, at least at the time of use, and
thereby form a complex containing the two enzymes linked together otherwise than through the target or through a whole antibody which binds directly to a target cell.

17. Use of at least one vehicle, at least one enzyme and linking means all as defined in any one of claims 1 to 13, to prepare a product for topical application.

18. A process for making a product for attacking a target, comprising incorporating into a vehicle distributing among a plurality of vehicles:
i) at least one of two enzymes which are an enzyme for generating an agent active against a target and a second enzyme for generating an intermediate which is a substrate for the first enzyme,
ii) linking means attached or attachable to both enzymes to couple the enzymes to each other, at least at the time of use, and
thereby form a complex containing the two enzymes linked together otherwise than through the target or through a whole antibody which binds directly to a target cell.

## Patentansprüche

1. Ein Produkt, enthaltend einen oder mehrere Träger, enthaltend, in dem gleichen Träger oder verteilt zwischen einer Vielzahl von Trägern:
(i) Zumindest eines der zwei Enzyme, die ein Enzym zur Erzeugung eines aktiven Mittels gegen ein Target sind, und ein zweites Enzym zur Erzeugung eines Zwischenprodukts, welches ein Substrat für das erste Enzym ist,
(ii) Bindungsmittel, verknüpft oder verknüpfbar mit beiden Enzymen zur Verbindung der Enzyme aneinander, zumindest für den Zeitraum der Verwendung, und
dadurch Bilden eines Komplexes, enthaltend die zwei Enzyme miteinander anders verbunden als durch das Target oder durch einen ganzen Antikörper, welcher direkt an einer Targetzelle bindet.

2. Ein Produkt nach Anspruch 1, worin das erste Enzym eine Oxidase ist, welche Wasserstoffperoxid erzeugt, und das zweite Enzym eine Peroxidase ist.

3. Ein Produkt nach Anspruch 1 oder Anspruch 2, enthaltend beide Enzyme.

4. Ein Produkt nach einem der Ansprüche 1 bis 3, worin das Bindungsmittel ein oder mehrere Antikörper oder Antikörperfragmente enthält, mit welchen die Enzyme verbunden sind, zumindest zum Zeitpunkt der Verwendung.

5. Ein Produkt nach Anspruch 4, worin jedes Enzym mit einem entsprechenden Antikörper verbunden ist und die Antikörper miteinander durch Antikörper-Antigen-Bindung verbunden sind.

6. Ein Produkt nach Anspruch 5, worin zumindest ein Enzym chemisch zu seinem entsprechenden Antikörper konjugiert ist.

7. Ein Produkt nach Anspruch 4, worin das Bindungsmittel zumindest ein Antikörperfragment enthält, an welches ein Enzym gebunden ist, zumindest zum Zeitpunkt der Verwendung, und das fähig ist, sich mit dem anderen Enzym durch Antikörper-Antigen-Bindung zu verbinden.

8. Ein Produkt nach einem der Ansprüche 1 bis 3, worin das Bindungsmittel ein Trägermaterial ist, an welches beide Enzyme chemisch gebunden sind.

9. Ein Produkt nach Anspruch 8, worin das Trägermaterial Polyethylenimin ist.

10. Ein Produkt nach einem der Ansprüche 1 bis 9, das auch einen Antikörper oder ein Antikörperfragment enthält, welches mit dem Komplex, enthaltend das Bindungsmittel und die zwei Enzyme, verbunden oder verbindbar ist, und das fähig ist, sich an die Targetstelle zu binden.

11. Ein Produkt nach Anspruch 10, worin die Verbindung des erwähnten Komplexes an den Antikörper oder an das Antikörperfragment, fähig, sich an die Targetstelle zu binden, durch Antikörper-Antigen-Bindung erfolgt.

12. Ein Produkt, enthaltend einen oder mehrere Träger, enthaltend, in dem gleichen Träger oder verteilt zwischen einer Vielzahl von Trägern:
(i) Zumindest eines der zwei Enzyme, die ein Enzym zur Erzeugung eines aktiven Mittels gegen ein Target sind, und ein zweites Enzym zur Erzeugung eines Zwischenprodukts, welches ein Substrat für das erste Enzym ist,
(ii) Bindungsmittel, verknüpft oder verknüpfbar mit beiden Enzymen zur Verbindung der Enzyme aneinander, zumindest für den Zeitraum der Verwendung, und
(iii) ein Antikörperfragment, welches verbunden oder verbindbar mit dem Komplex ist, enthaltend die Bindungsmittel und die zwei Enzyme, und welches fähig ist, sich an die Targetstelle zu binden.

13. Ein Produkt nach einem der vorstehenden Ansprüche, worin das Target eine orale bakterielle Spezies ist und der oder jeder der Träger geeignet ist, in den Mund genommen zu werden.

14. Ein Produkt nach Anspruch 13, welches eine Zahnpasta, ein Mundwasser oder eine Rautenpastille ist, oder in welchem zumindest ein Träger dieser Art vorgesehen ist.

15. Ein kosmetisches Verfahren des Angreifens eines Targets, welches die Lieferung eines Produktes in dessen Nachbarschaft gemäß einem der vorstehenden Ansprüche umfaßt.

16. Ein kosmetisches Verfahren des Angreifens eines Targets, welches umfaßt die Lieferung in die Nachbarschaft desselben von
(i) zumindest einem der zwei Enzyme, die ein Enzym zur Erzeugung eines aktiven Mittels gegen ein Target sind, und einem zweiten Enzym zur Erzeugung eines Zwischenprodukts, welches ein Substrat für das erste Enzym ist,
(ii) Bindungsmitteln, verknüpft oder verknüpfbar mit beiden Enzymen zur Verbindung der Enzyme aneinander, zumindest für den Zeitraum der Verwendung, und
dadurch einen Komplex bildet, enthaltend die zwei Enzyme, verbunden zusammen anders als durch das Target oder durch einen ganzen Antikörper, welcher direkt an einer Targetzelle bindet.

17. Verwendung von zumindest einem Träger, zumindest einem Enzym und Bindungsmittel, alle wie sie in irgendeinem der Ansprüche 1 bis 13 definiert sind, zur Herstellung eines Produkts für lokale Anwendung.

18. Verfahren zur Herstellung eines Produkts zum Angreifen eines Targets, umfassend das Inkorporieren in einen Träger, verteilend unter eine Vielzahl von Trägern von
(i) zumindest einem der zwei Enzyme, die ein Enzym zur Erzeugung eines aktiven Mittels gegen ein Target sind, und einem zweiten Enzym zur Erzeugung eines Zwischenprodukts, welches ein Substrat für das erste Enzym ist,
(ii) Bindungsmitteln, verknüpft oder verknüpfbar mit beiden Enzymen zur Verbindung der Enzyme aneinander, zumindest für den Zeitraum der Verwendung, und
dadurch einen Komplex bildet, enthaltend die zwei Enzyme, verbunden zusammen anders als durch das Target oder durch einen ganzen Antikörper, welcher direkt an einer Targetzelle bindet.

## Revendications

1. Produit comprenant un ou plusieurs véhicules contenant, dans le même véhicule ou réparti entre plusieurs véhicules:
i) au moins une des deux enzymes qui sont une enzyme pour engendrer un agent actif contre une cible et une seconde enzyme pour engendrer un intermédiaire qui est un substrat pour la première enzyme,
ii) un moyen de liaison fixé ou fixable aux deux enzymes pour coupler les enzymes l'une à l'autre, au moins au moment de l'emploi, et former ainsi un complexe contenant les deux enzymes liées ensemble autrement que par l'intermédiaire de la cible ou par l'intermédiaire d'un anticorps entier qui se lie directement à une cellule cible.

2. Produit selon la revendication 1, dans lequel la première enzyme est une oxydase qui engendre du peroxyde d'hydrogène et la seconde enzyme est une peroxydase.

3. Produit selon la revendication 1 ou 2, comprenant 2 enzymes.

4. Produit selon l'une quelconque des revendications 1 à 3, dans lequel le moyen de liaison comprend 1 ou plusieurs anticorps ou fragment d'anticorps auquel les enzymes sont unies au moins au moment de l'emploi.

5. Produit selon la revendication 4, dans lequel chaque enzyme est uni à un anticorps respectif et les anticorps se lient l'un à l'autre par une liaison anticorps-antigène.

6. Produit selon la revendication 5, dans lequel au moins une enzyme est chimiquement conjugué à son anticorps respectif.

7. Produit selon la revendication 4, dans lequel le moyen le moyen de liaison contient au moins un fragment d'anticorps auquel une enzyme est fixée, au moins au moment de l'emploi, et qui est capable de se lier à l'autre enzyme par l'intermédiaire d'une liaison anticorps-antigène.

8. Produit selon l'une quelconque des revendications 1 à 3, dans lequel le moyen de liaison est un matériau porteur auquel les deux enzymes sont chimiquement conjuguées.

9. Produit selon la revendication 8, dans lequel le matériau porteur est une polyéthylèneimine.

10. Produit selon l'une quelconque des revendications 1 à 9 contenant également un anticorps ou un fragment d'anticorps qui est fixé ou fixable au complexe comprenant le moyen de liaison et les deux enzymes et qui est capable de se lier au site cible.

11. Produit selon la revendication 10, dans lequel la fixation dudit complexe à l'anticorps ou au fragment d'anticorps capable de se lier au site cible est réalisé par l'intermédiaire d'une liaison anticorps-antigène.

12. Produit comprenant un ou plusieurs véhicules contenant, dans le même véhicule ou réparti entre plusieurs véhicule:
i) au moins une des deux enzyme qui sont une enzyme pour engendrer un agent actif contre une cible et une seconde enzyme pour engendrer un intermédiaire qui est un substrat pour la première enzyme,
ii) un moyen de liaison fixé ou fixable aux deux enzymes pour coupler les enzymes l'une à l'autre, au moins au moment de l'emploi, et
iii) un fragment d'anticorps qui est fixé ou fixable au complexe comprenant le moyen de liaison et les deux enzymes et qui est capable de se lier au site cible.

13. Produit selon l'une quelconque des revendications précédentes, dans lequel la cible est une espèce bactérienne orale et le ou chacun desdits véhicules est acceptable pour être pris dans la bouche.

14. Produit selon la revendication 13 qui est, ou dans lequel au moins un véhicule est fourni par, une pâte dentifrice, un bain de bouche ou une pastille.

15. Procédé cosmétique de fixation d'une cible qui comprend la délivrance à sa proximité d'un produit conformément à l'une quelconque des revendications précédentes.

16. Procédé cosmétique de fixation d'une cible qui comprend la délivrance à sa proximité
i) au moins une des deux enzymes qui sont une enzyme pour engendrer un agent actif contre une cible, et une seconde enzyme pour engendrer un intermédiaire qui est un substrat pour la première enzyme, et
ii) un moyen de liaison fixé ou fixable au deux enzymes pour coupler les deux enzymes l'une à l'autre au moins au moment de l'emploi, et
former ainsi un complexe contenant les deux enzymes liées ensemble autrement que par l'intermédiaire de la cible par l'intermédiaire d'un anticorps entier qui le lie directement à une cellule cible.

17. Utilisation d'au moins un véhicule, d'au moins une enzyme et d'un moyen de liaison tous tels que définis dans l'une quelconque des revendications 1 à 13, pour préparer un produit pour une application topique.

18. Procédé pour fabriquer un produit destiné à être fixé à une cible, comprenant l'incorporation dans un véhicule ou la répartition parmi plusieurs véhicules:
i) d'au moins une des deux enzymes qui sont une enzyme pour engendrer un agent actif contre une cible et une seconde enzyme pour engendrer un intermédiaire qui est un substrat pour la première enzyme,
ii) d'un moyen de liaison fixé ou fixable aux deux enzymes pour coupler les enzymes l'une à l'autre, au moins au moment de l'emploi, et
former ainsi un complexe contenant les deux enzymes liées ensemble autrement que par l'intermédiaire de la cible ou par l'intermédiaire d'un anticorps entier qui se lie directement à une cellule cible.
